# EUROPÄISCHE PATENTSCHRIFT

(11) **EP 2 041 060 B1**
(45) Veröffentlichungstag und Bekanntmachung des Hinweises auf die Patenterteilung: **16.12.2009**
(21) Anmeldenummer: 07786855.2
(22) Anmeldetag: 27.06.2007
(51) Int. Cl.: C07C 45/28, C07C 49/413, C07C 49/607

(54) **VERFAHREN ZUR HERSTELLUNG VON CYCLISCHEN KETONEN**
METHOD FOR THE PRODUCTION OF CYCLIC KETONES
PROCÉDÉ DE PRODUCTION DE CÉTONES CYCLIQUES

(30) Priorität: 29.06.2006 EP 06116261
(43) Veröffentlichungstag der Anmeldung: 01.04.2009
(73) Patentinhaber: BASF SE, 67056 Ludwigshafen (DE)
(72) Erfinder: TELES, Joaquim Henrique, 67166 Otterstadt (DE); RÖSSLER, Beatrice, 67256 Weisenheim am Sand (DE); PINKOS, Rolf, 67098 Bad Dürkheim (DE); TEBBEN, Gerd, 68159 Mannheim (DE); MÜLLER, Christian, 68167 Mannheim (DE)
(86) Internationale Anmeldenummer: PCT/EP2007/056396
(87) Internationale Veröffentlichungsnummer: WO 2008/000757

(56) Entgegenhaltungen:
- WO-A-2005/030689
- WO-A-2005/030690
- US-A1- 2006 106 258
- DATABASE WPI Week 198531 Derwent Publications Ltd., London, GB; AN 1985-188542 XP002414853 & SU 1 133 257 A (FALKOVICH M I) 7. Januar 1985 (1985-01-07)
- STAROKON E V ET AL: "LIQUID PHASE OXIDATION OF ALKENES WITH NITROUS OXIDE TO CARBONYL COMPOUNDS" ADVANCED SYNTHESIS AND CATALYSIS, WILEY-VCH, WEINHEIM, DE, Bd. 346, Nr. 2-3, 2004, Seiten 268-274, XP002321384 ISSN: 1615-4150 in der Anmeldung erwähnt

## Beschreibung

Die vorliegende Erfindung betrifft ein Verfahren zur Herstellung eines cyclischen Ketons mit 7 bis 16 C-Atomen, mindestens umfassend die Oxidation einer Zusammensetzung (I), mindestens enthaltend ein cyclisches Olefin mit 7 bis 16 C-Atomen mit mindestens einer C-C-Doppelbindung, mittels Distickstoffmonoxid unter Erhalt einer Zusammensetzung (A), und das Behandeln der Zusammensetzung (A) mit mindestens einer Base unter Erhalt einer Zusammensetzung (B).

Die Oxidation einer olefinischen Verbindung zu einem Aldehyd oder einem Keton mittels Distickstoffmonoxid ist beispielsweise beschrieben in der GB 649,680 oder der dazu äquivalenten US 2,636,898. In beiden Schriften wird ganz allgemein offenbart, dass die Oxidation prinzipiell in Anwesenheit eines geeigneten Oxidationskatalysators erfolgen kann.

In den neueren wissenschaftlichen Artikeln von G. I. Panov et al., "Non-Catalytic Liquid Phase Oxidation of Olefines with Nitrous Oxide. 1. Oxidation of Cyclohexene to Cyclohexanone", React. Kinet. Catal. Lett. Vol. 76, No. 2 (2002) S. 401-405, und K. A. Dubkov et al., "Non-Catalytic Liquid Phase Oxidation of Olefines with Nitrous Oxide. 2. Oxidation of Cyclopentene to Cyclopentanone", React. Kinet. Catal. Lett. Vol. 77, No. 1 (2002) S. 197-205 werden ebenfalls Oxidationen von olefinischen Verbindungen mit Distickstoffmonoxid beschrieben. Auch ein wissenschaftlicher Artikel "Liquid Phase Oxidation of Olefines with Nitrous Oxide to Carbonyl Compounds" von E. V. Starokon et al. in Adv. Synth. Catal. 2004, 346, 268 - 274 beinhaltet eine mechanistische Studie der Oxidation von Olefinen mit Distickstoffmonoxid in flüssiger Phase.

Die Synthese von Carbonylverbindungen aus Olefinen mit Distickstoffmonoxid wird auch in verschiedenen internationalen Patentanmeldungen beschrieben. So offenbart die WO 03/078370 ein Verfahren zur Herstellung von Carbonylverbindungen aus aliphatischen Olefinen mit Distickstoffmonoxid. Die Umsetzung wird bei Temperaturen im Bereich von 20 bis 350°C und Drücken von 0,01 bis 100 atm durchgeführt. Die WO 03/078374 offenbart ein entsprechendes Verfahren zur Herstellung von Cyclohexanon. Gemäß der WO 03/078372 werden cyclische Ketone mit 4 bis 5 C-Atomen hergestellt. Gemäß der WO 03/078375 werden unter diesen Verfahrensbedingungen cyclische Ketone aus cyclischen Olefinen mit 7 bis 20 C-Atomen hergestellt. WO 03/078371 offenbart ein Verfahren zur Herstellung substituierter Ketone aus substituierten Olefinen. WO 04/000777 offenbart ein Verfahren zur Umsetzung von-Di- und PolyOlefinen mit Distickstoffmonoxid zu den entsprechenden Carbonylverbindungen.

In WO 2005/030690 und WO 2005/030689 werden Verfahren zur Herstellung von Cyclododecanon beschrieben, wobei in einem Verfahrensschritt eine Oxidation mit Distickstoffmonoxid erfolgt. In WO 2005/030690 wird ein Verfahren zur Herstellung von Cyclododecanon durch Oxidation von 1,5,9-Cyclododecatrien (CDT) mit N₂O zu Cyclododeca-4,8-dienon und anschließende Hydrierung von Cyclododeca-4,8-dienon zu Cyclododecanon beschrieben.

Allen Verfahren gemeinsam ist, dass die Reinheit der Rohprodukte ohne zusätzliche Reinigung für einige Anwendungen nicht ausreicht. Insbesondere organische Verbindungen mit sauerstoffhaltigen Gruppen sind häufig noch in zu hohen Mengen in den erhaltenen Produkten enthalten.

Bei der Oxidation von Olefinen mittels Distickstoffmonoxid können sich beispielsweise Aldehyde als Nebenprodukte bilden, wie beispielsweise in Panov et al., Adv. Synth. Catal. (2004) 346, 268-274 beschrieben.

Dies ist insofern problematisch als cyclische Ketone für verschiedene Anwendungen in hoher Reinheit benötigt werden. So ist beispielsweise Cyclododecanon ein wichtiges Zwischenprodukt für die Herstellung von beispielsweise Laurinlactam, Dodecandicarbonsäure und daraus abgeleiteten Polyamide wie beispielsweise Nylon 12 oder Nylon 6.12. Dabei können die in den cyclischen Ketonen enthaltenen Verunreinigungen wie Aldehyde durch herkömmliche Reinigungsverfahren wie Destillation, Extraktion oder Umkristallisation nur schwer entfernt werden, da die funktionellen Gruppen und die Anzahl der Kohlenstoffatome ähnlich ist. Deshalb ist in diesen Fällen eine sehr aufwendige Reinigung, beispielsweise durch vielstufige Destillation und/oder Kristallisation notwendig. Diese Reinigungsverfahren sind dadurch aufwendig und kostenintensiv.

Der vorliegenden Erfindung lag daher die Aufgabe zu Grunde, ein Verfahren bereit zu stellen, mit dem cyclische Ketone einfach und mit geringem Aufwand in hoher Reinheit erhalten werden können.

Erfindungsgemäß wird diese Aufgabe gelöst durch ein Verfahren zur Herstellung eines cyclischen Ketons mit 7 bis 16 C-Atomen, mindestens umfassend die Stufen
(a) Oxidation einer Zusammensetzung (I), mindestens enthaltend ein cyclisches Olefin mit 7 bis 16 C-Atomen mit mindestens einer C-C-Doppelbindung, mittels Distickstoffmonoxid unter Erhalt einer Zusammensetzung (A),
(b) Behandeln der Zusammensetzung (A) mit mindestens einer Base unter Erhalt einer Zusammensetzung (B).

Es wurde nun überraschend gefunden, dass insbesondere Nebenprodukte wie cyclische und offenkettige Aldehyde allgemein aus einem Gemisch mit cyclischen Ketonen mit derselben oder einer ähnlichen Anzahl an C-Atomen selektiv abgereichert werden können, indem man das Gemisch mit einer Base, beispielsweise mit einer Base bei erhöhter Temperatur, behandelt. Unter dem Begriff "abgereichert" wird im Rahmen der vorliegenden Erfindung verstanden, dass die Menge des Aldehyds im Verhältnis zum cyclischen Keton reduziert wird, und insbesondere das cyclische Keton im Wesentlichen nicht angegriffen wird.

Unter "Behandeln" wird im Rahmen der vorliegenden Anmeldung ein in Kontakt bringen der Zusammensetzung mit mindestens einer Base verstanden.

Durch das erfindungsgemäße Verfahren können cyclische Ketone mit einer Reinheit von beispielsweise > 99,5 % erhalten werden. Dabei kann das erfindungsgemäße Verfahren leicht mit bestehenden Anlagen kombiniert werden, so dass keine kostenintensiven Umbauten erforderlich sind. Ferner stellt das erfindungsgemäße Verfahren eine Möglichkeit dar, bei Oxidationen von Olefinen mit Distickstoffmonoxid die Ausbeute an cyclischen Ketonen zu verbessern, da die Behandlung mit Base im Allgemeinen sehr selektiv ist und damit weniger Produkt verloren geht.

Die Umsetzung gemäß Stufe (a) kann generell gemäß sämtlicher Verfahrensführungen erfolgen, bei denen das Olefin und Distickstoffmonoxid miteinander reagieren

Gemäß Stufe (a) des erfindungsgemäßen Verfahrens wird das cyclische Olefin durch Umsetzung mit Distickstoffmonoxid oxidiert. Dabei kann für die Umsetzung des cyclischen Olefins mit Distickstoffmonoxid mindestens ein geeignetes Lösungsmittel oder Verdünnungsmittel eingesetzt werden. Als solche sind unter anderem cyclische Alkane, beispielsweise Cyclododecan oder Cyclododecanon oder gesättigte aliphatische oder aromatische, gegebenenfalls alkylsubstituierte Kohlenwasserstoffe zu nennen, wobei im Wesentlichen alle gängigen Lösungs- und/oder Verdünnungsmittel geeignet sind unter der Maßgabe, dass sie weder eine C-C-Doppelbindung, noch eine C-C-Dreifachbindung, noch eine Aldehydgruppe aufweisen.

Im Allgemeinen ist bei der Umsetzung des cyclischen Olefins mit Distickstoffmonoxid der Zusatz eines Lösungsmittels oder Verdünnungsmittel nicht notwendig.

Die Temperaturen bei der Umsetzung des cyclischen Olefins mit Distickstoffmonoxid liegen bevorzugt im Bereich von 140 bis 350°C, weiter bevorzugt im Bereich von 180 bis 320°C und besonders bevorzugt im Bereich von 200 bis 300°C.

Es ist möglich, die Umsetzung des cyclischen Olefins mit Distickstoffmonoxid bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden.

Die Drücke bei der Umsetzung des cyclischen Olefins mit Distickstoffmonoxid liegen bevorzugt höher als der Eigendruck des Edukt- bzw. Produktgemisches bei der gewählten Reaktionstemperatur oder den gewählten Reaktionstemperaturen. Die Drücke liegen bevorzugt im Bereich von 1 bis 1000 bar, weiter bevorzugt im Bereich von 40 bis 300 bar und besonders bevorzugt im Bereich von 50 bis 200 bar.

Der Druck im Reaktionsgefäß, bevorzugt in mindestens einem Rohrreaktor, liegt im Allgemeinen bei Werten, die größer oder gleich, bevorzugt größer dem Eigendruck des Eduktgemisches beziehungsweise des Produktgemisches bei der gewählten Reaktionstemperatur oder den gewählten Reaktionstemperaturen im Reaktionsgefäß sind. Im Allgemeinen liegen die Reaktionsdrücke im Bereich von 1 bis 14000 bar, bevorzugt im Bereich vom Eigendruck bis 3000 bar, besonders bevorzugt im Bereich vom Eigendruck bis 1000 bar und insbesondere bevorzugt im Bereich vom Eigendruck bis 325 bar, beispielsweise bei 50 bis 200 bar.

Es ist möglich, die Umsetzung des cyclischen Olefins mit Distickstoffmonoxid bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden.

Hinsichtlich der für die Umsetzung des cyclischen Olefins mit Distickstoffmonoxid einsetzbaren Reaktoren existieren keine besonderen Beschränkungen. Insbesondere kann die Umsetzung in Batch-Fahrweise oder in kontinuierlicher Fahrweise erfolgen. Demgemäß können beispielsweise als Reaktoren mindestens ein CSTR (Continuous Stirred Tank Reactor) mit mindestens einem internen und/oder mindestens einem externen Wärmetauscher, mindestens ein Rohrreaktor, mindestens einem Rohrbündelreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Bevorzugt wird die Umsetzung des cyclischen Olefins mit Distickstoffmonoxid in einem einzigen Reaktor durchgeführt. Beispielsweise bevorzugt ist die Umsetzung in kontinuierlicher Fahrweise.

Die Verweilzeit des Reaktionsgutes in dem mindestens einen Reaktor bei der Umsetzung des cyclischen Olefins mit Distickstoffmonoxid liegt im allgemeinen im Bereich von bis zu 20 h, bevorzugt im Bereich von 0,1 bis 20 Stunden, weiter bevorzugt im Bereich von 0,2 bis 15 Stunden und besonders bevorzugt im Bereich von 0,25 bis 10 h.

Im Feed, der der Umsetzung von Distickstoffmonoxid mit dem cyctischen Olefin zugeführt wird, liegt das Molverhältnis von Distickstoffmonoxid und dem cyclischen Olefin im Allgemeinen im Bereich von 0,05 bis 4, bevorzugt im Bereich von 0,06 bis 1, weiter bevorzugt im Bereich von 0,07 bis 0,5 und besonders bevorzugt im Bereich von 0,1 bis 0,4.

Die Umsetzung des cyclischen Olefins mit Distickstoffmonoxid kann so durchgeführt werden, dass bei einer sehr hohen Selektivität bezüglich des cyclischen Ketons ein Umsatz des cyclischen Olefins im Bereich von bis zu 50 %, bevorzugt im Bereich von 5 bis 30 % und insbesondere bevorzugt im Bereich von 10 bis 20 % erreicht wird. Dabei liegt die Selektivität, bezogen auf das cyclische Keton, im Allgemeinen bei mindestens 90 %, bevorzugt bei mindestens 92,5 % und besonders bevorzugt bei mindestens 93 %.

Grundsätzlich kann erfindungsgemäß jedes cyclische Olefin mit 7 bis 16 C-Atomen oder jedes Gemisch aus zwei und mehr unterschiedlichen cyclischen Olefinen mit 7 bis 16 C-Atomen mit Distickstoffmonoxid umgesetzt werden. Geeignete Olefine sind beispielsweise cyclische Olefine mit einer oder mehreren C-C-Doppelbindungen. Weiter bevorzugt sind cyclische Olefine mit einer oder mehreren C-C-Doppelbindungen wie beispielsweise Cyclohepten, Cycloocten, Cyclodecen, Cycloundecen, Cyclododecen, 1,5-Cyclooctadien, 1,5-Cyclododecadien oder 1,5,9-Cyclododecatrien.

Besonders bevorzugt wird als Olefin Cyclododecen oder 1,5,9-Cyclododecatrien eingesetzt. Unter anderem sind hierbei beispielsweise 1,5,9-Cyclododecatriene, beispielsweise cis,trans,trans-1,5,9-Cyclododecatrien oder cis,cis,trans-1,5,9-Cyclododecatrien oder all-trans-1,5,9-Cyclododecatrien, zu nennen.

Bevorzugt wird als Cyclododecatrien cis,trans,trans-1,5,9-Cyclododecatrien eingesetzt.

Daher betrifft die vorliegende Erfindung in einer bevorzugten Ausführungsform ein Verfahren zur Herstellung eines Ketons wie oben beschrieben, wobei das Olefin ausgewählt ist aus der Gruppe bestehend aus Cyclododecen und Cyclododecatrien, insbesondere 1,5,9-Cyclododecatrien.

Die Umsetzung des cyclischen Olefins mit Distickstoffmonoxid kann grundsätzlich in Anwesenheit eines Katalysators erfolgen, jedoch auch ohne Zusatz eines Katalysators.

Im Rahmen der vorliegenden Erfindung kann Distickstoffmonoxid in reiner Form oder in Form eines Gasgemisches enthaltend Distickstoffmonoxid eingesetzt werden.

Grundsätzlich kann in Stufe (a) des erfindungsgemäßen Verfahrens jedes Gasgemisch enthaltend Distickstoffmonoxid eingesetzt werden. Erfindungsgemäß ist es auch möglich, das Gasgemisch enthaltend Distickstoffmonoxid vor Einsatz in die Umsetzung gemäß Stufe (a) zu Reinigen oder Aufzukonzentrieren. Ein geeignetes Reinigungsverfahren umfasst beispielsweise die Absorption des Gasgemisches in einem organischen Lösungsmittel oder Wasser, die Desorption des Gasgemisches aus dem beladenen organischen Lösungsmittel oder dem beladenen Wasser und das Einstellen des Gehalts an Stickoxiden NOₓ in dem Gasgemisch auf höchstens 0,01 bis 0,001 Vol.-%, bezogen auf das Gesamtvolumen des Gasgemisches. Ein derartiges Verfahren ist beispielsweise in DE 10 2004 046 167.8 beschrieben, deren diesbezüglicher Inhalt vollumfänglich in den Kontext der vorliegenden Anmeldung aufgenommen wird.

Dabei kann das eingesetzte Gasgemisch enthaltend Distickstoffmonoxid grundsätzlich aus jeder beliebigen Quelle stammen. Insbesondere ist es möglich, dass als Distickstoffmonoxidquelle das Abgas eines Verfahrens eingesetzt wird wie in DE 10 2004 046 167.8 beschrieben.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig, und wird im Rahmen der vorliegenden Erfindung weiter als Gasgemisch bezeichnet.

Erfindungsgemäß kann auch ein Gemisch verschiedener Abgase eingesetzt werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandisäureanlage, einer Hydroxylaminanlage und/oder einer Salpetersäureanlage, wobei letztere wiederum bevorzugt mit mindestens einem Abgas einer Adipinsäureanlage, einer Dodecandisäureanlage oder einer Hydroxylaminanlage betrieben wird.

Erfindungsgemäß kann das Gasgemisch gasförmig eingesetzt werden. Es ist jedoch auch möglich, das Gasgemisch enthaltend Distickstoffmonoxid zunächst derart zu behandeln, dass das Gasgemisch in flüssiger oder überkritischer Form vorliegt und dann eingesetzt wird. Das Gasgemisch bzw. Distickstoffmonoxid kann durch geeignete Wahl des Drucks oder der Temperatur verflüssigt werden. Ebenso ist es im Rahmen der vorliegenden Erfindung möglich, das Gasgemisch in ein Lösungsmittel einzulösen.

Erfindungsgemäß enthält die Zusammensetzung (I) das cyclische Olefin üblicherweise in einer Menge von mehr als 80 Gew.%, vorzugsweise 85 bis 99,999 Gew.%, insbesondere 90 bis 99,99 Gew.%, besonders bevorzugt 92 bis 99,9 Gew.%, beispielsweise 95 bis 99,8 Gew.%. Die Zusammensetzung (I) kann neben dem cyclischen Olefin üblicherweise weitere Verbindungen, insbesondere organische Verbindungen, enthalten.

Die Zusammensetzung (I) kann als organische Verbindungen beispielsweise Vinylcyclohexen, Cyclooctadien und beispielsweise bicyclische Isomere des 1,5,9-Cyclododecatriens enthalten. Weiterhin ist es möglich, dass Spuren an C₁₆-Komponenten, C₂₄-Komponenten oder höheren Oligomeren enthalten sind.

Bei der Umsetzung gemäß Stufe (a) des erfindungsgemäßen Verfahrens wird das in der Zusammensetzung (I) enthaltene cyclische Olefin mittels Distickstoffmonoxid oxidiert. Dabei wird eine Zusammensetzug (A) enthaltend mindestens ein cyclisches Keton erhalten.

Die Zusammensetzung (A) enthält im Rahmen der vorliegenden Erfindung mindestens ein cyclisches Keton mit 7 bis 16 C-Atomen. Erfindungsgemäß enthält die Zusammensetzung (A) das cyclische Keton in einer Menge von mehr als 5 Gew.-%, bevorzugt mehr als 10 Gew.%, vorzugsweise 10 bis 90 Gew.%, insbesondere 11 bis 50 Gew.%, besonders bevorzugt 12 bis 40 Gew.%, insbesondere bevorzugt 13 bis 30 Gew.%, beispielsweise 14 bis 20 Gew.% oder 15 bis 18 Gew.%. Die Zusammensetzung (A) enthält neben dem cyclischen Olefin üblicherweise weitere Verbindungen, insbesondere organische Verbindungen, beispielsweise nicht umgesetztes Edukt oder organische Verbindungen mit sauerstoffhaltigen Gruppen, beispielsweise Alkohole, Aldehyde oder Epoxide. Dabei können die organischen Verbindungen insbesondere dieselbe Anzahl an C-Atomen aufweisen, wie das in der Zusammensetzung (A) enthaltene cyclische Keton.

Die Zusammensetzung (A) kann direkt in Stufe (b) des erfindungsgemäßen Verfahrens eingesetzt werden. Es ist jedoch auch möglich, dass die Zusammensetzung (A) vor Stufe (b) einer Zwischenbehandlung unterzogen wird. Beispielsweise kann nicht umgesetztes Edukt aus der Zusammensetzung (A) abgetrennt werden. Ebenso ist es möglich, dass Nebenprodukte der Oxidation, beispielsweise Diketone, abgetrennt werden. Erfindungsgemäß können vorzugsweise nicht umgesetztes Edukt und Diketone abgetrennt werden. Die Abtrennung kann beispielsweise destillativ erfolgen, wobei die Destillation in einer oder mehreren Kolonnen durchgeführt wird, vorzugsweise in mindestes zwei Kolonnen.

Erfindungsgemäß wird die Zusammensetzung (A) gemäß Stufe (b) mit mindestens einer Base behandelt. Dabei sind die Verfahrensbedingungen der Behandlung in weiten Bereichen variierbar, solange gewährleistet ist, dass die Konzentration mindestens einer störenden Nebenkomponente, insbesondere mindestens eines Aldehyds verringert wird.

Erfindungsgemäß wird dabei die Menge des Aldehyds verringert, wobei das cyclische Keton im wesentlichen nicht angegriffen wird. Dabei werden erfindungsgemäß offenkettige Aldehyde vorzugsweise zu mehr als 90% abgebaut, insbesondere zu mehr als 95 % und besonders bevorzugt zu 99,99%. Cyclische Aldehyde mit exocyclischer Aldehydgruppe werden vorzugsweise zu bis zu 30% abgebaut, insbesondere zu bis zu 35 % und besonders bevorzugt zu bis zu 40%. Erfindungsgemäß wird das cyclische Keton nur zu 0,5 bis 2,0 %, vorzugsweise zu 0,75 bis 1,75 %, insbesondere zu 1,0 bis 1,5 % abgebaut.

In der Regel wird die Behandlung mit Base so lange fortgeführt bis mindestens 90%, bevorzugt mindestens 95% der störenden Nebenkomponente, insbesondere des mindestens einen Aldehyds, vorzugsweise des mindestens einen offenkettigen Aldehyds, umgesetzt sind.

Erfindungsgemäß erfolgt das Behandeln mit mindestens einer Base gemäß Stufe (b) vorzugsweise für einen Zeitraum von 1 Minute bis 10 Stunden, insbesondere 5 Minuten bis 5 Stunden, besonders bevorzugt 10 bis 60 Minuten, weiter bevorzugt 20 bis 50 Minuten.

Dabei kann das Behandeln im Rahmen des erfindungsgemäßen Verfahrens insbesondere bei einer Temperatur von 100 bis 250°C erfolgen, bevorzugt 110 bis 220°C, besonders bevorzugt 120 bis 200°C, weiter bevorzugt 150 bis 190°C.

Daher betrifft die vorliegende Erfindung gemäß einer weiteren Ausführungsform auch ein wie zuvor beschriebenes Verfahren zur Herstellung eines cyclischen Ketons mit 7 bis 16 C-Atomen, wobei die Behandlung gemäß Stufe (b) bei einer Temperatur von 100 bis 250°C und für einen Zeitraum von 1 Minute bis 10 Stunden erfolgt.

Für die Behandlung mit der Base sind alle möglichen Reaktortypen geeignet. Für eine kontinuierliche Reaktionsführung werden bevorzugt Reaktoren mit einer Rohrcharakteristik, wie z.B. Rohrreaktoren, Rührkesselkaskaden oder vergleichbare Reaktoren eingesetzt. Für eine diskontinuierliche Reaktionsführung (Batch-Verfahren) sind einfache Rührkessel gut geeignet. Vorzugsweise läuft die Reaktion im wesentlichen homogen in der Flüssigphase ab.

Vorzugsweise umfasst die Behandlung gemäß Stufe (b) zwei Teilschritte (b1) und (b2), wobei gemäß Schritt (b1) die Zusammensetzung (A) mit mindestens einer Base behandelt wird und gemäß Schritt (b2) die Base abgetrennt wird.

Daher betrifft die vorliegende Erfindung gemäß einer weiteren Ausführungsform auch ein wie zuvor beschriebenes Verfahren zur Herstellung eines cyclischen Ketons mit 7 bis 16 C-Atomen, wobei die Stufe (b) die Teilschritte (b1 ) und (b2) umfasst:
(b1) Behandeln der Zusammensetzung (A) mit mindestens einer Base
(b2) Abtrennung der Base.

Die Abtrennung der Base gemäß (b2) kann gemäß allen üblichen Verfahren erfolgen, beispielsweise durch Destillation. Insbesondere wenn als Base NaOH oder KOH eingesetzt werden, erfolgt die Abtrennung bevorzugt durch Verdampfung, zum Beispiel in Form eines Fallfilmverdampfers eines wiped film evaporators oder Wendelrohrverdampfers, oder durch Extraktion der Base, beispielsweise mit Wasser.

Im Rahmen der vorliegenden Erfindung können prinzipiell alle geeigneten Basen eingesetzt werden. Vorzugsweise werden organische oder anorganische Base eingesetzt, deren konjugierte Säure einen pKₐ relativ zu Wasser von >9 haben. Bevorzugt sind im Rahmen der vorliegenden Erfindung beispielsweise Trialkylamine, Alkali- oder Erdalkalialkoholate und Tetraalkylammonium-, Alkali- oder Erdalkalihydroxide. Ganz besonders bevorzugt sind Natrium- und Kaliumhydroxid.

Daher betrifft die vorliegende Erfindung gemäß einer weiteren Ausführungsform auch ein wie zuvor beschriebenes Verfahren zur Herstellung eines cyclischen Ketons mit 7 bis 16 C-Atomen, wobei die Base ausgewählt ist aus Natriumhydroxid und Kaliumhydroxid.

Die Base kann erfindungsgemäß entweder als reine Substanz oder als Lösung eingesetzt werden. Flüssige Basen werden bevorzugt ohne den Zusatz eines Lösungsmittels eingesetzt. Feste Basen werden bevorzugt als Lösung eingesetzt. Als Lösungsmittel wird bevorzugt die konjugierte Säure eingesetzt. Die besonders bevorzugten Basen NaOH und KOH werden bevorzugt als konzentrierte wässrige Lösung eingesetzt. Vorzugsweise weist eine als Lösung eingesetzte Base eine Konzentration von mindestens 25 Gew.-%, insbesondere von mindestens 40 Gew.-%, besonders bevorzugt von etwa 50 Gew.-% auf.

Die Menge der gemäß Stufe (b) eingesetzten Base kann in weiten Bereichen variiert werden. Es können zwischen 0,01 und 5 mol Base/mol Aldehyd eingesetzt werden. Bevorzugt werden zwischen 0,05 und 2 mol Base/mol Aldehyd eingesetzt. Besonders bevorzugt zwischen 0,1 und 1 mol Base/mol Aldehyd eingesetzt.

Das Behandeln mit der Base wird im Temperaturbereich zwischen 100 und 250°C durchgeführt. Bevorzugt wird die Umsetzung zwischen 110 und 220 °C durchgeführt. Besonders bevorzugt wird die Umsetzung zwischen 150 und 190°C durchgeführt. Die Dauer der Behandlung wird durch die gewählte Temperatur, Basenart und -menge und durch den gewünschten Abreicherungsgrad für die Aldehyde bestimmt. Dabei werden die Bedingungen vorzugsweise so gewählt, dass die Dauer des Behandelns zwischen 1 Minute und 10 Stunden liegt, beispielsweise zwischen 10 Minuten und 5 Stunden, bevorzugt zwischen 20 Minuten und 2 Stunden, insbesondere zwischen 30 Minuten und 1,5 Stunden, besonders bevorzugt zwischen 40 Minuten und 1 Stunde.

Gemäß einer bevorzugten Ausführungsform wird das Behandeln mit der Base bei einer Temperatur von 160 bis 185°C für eine Zeit von 30 bis 40 Minuten durchgeführt. Vorzugsweise wird das Behandeln mit 0,1 bis 0,15 Gew.-% Natriumhydroxid, bezogen auf die gesamte Zusammensetzung, durchgeführt. Gemäß einer besonders bevorzugten Ausführungsform wird das Behandeln mit der Base bei einer Temperatur von 160 bis 185°C für eine Zeit von 30 bis 40 Minuten mit 0,1 bis 0,15 Gew.-% Natriumhydroxid, bezogen auf die gesamte Zusammensetzung, durchgeführt.

Erfindungsgemäß enthält die Zusammensetzung (B) mindestens ein cyclisches Keton. Dabei enthält die Zusammensetzung (B) das cyclische Keton üblicherweise in einer Menge von mehr als 40 Gew.%, vorzugsweise 50 bis 99,9 Gew insbesondere 55 bis 99 Gew.%, besonders bevorzugt 60 bis 95 Gew.%.

Die Zusammensetzung (B) kann beispielsweise auch weitere organische Verbindungen enthalten, beispielsweise 1,5,9-Cyclododecatrien, 1,2-Epoxycyclododeca-5,9-dien, Cycloundecadiencarbaldehyd, C₁₂-Diketone oder Spuren von oligomeren Verbindungen. Die Zusammensetzung (B) kann beispielsweise 1,5,9-Cyclododecatrien in Mengen von 0,2 bis 0,6 Gew.-%, 1,2-Epoxycyclododeca-5,9-dien in Mengen von 0,01 bis 0,1 Gew.-%, Cycloundecadiencarbaldehyd in Mengen von 0,5 bis 1,2 Gew.-%, C₁₂-Diketone in Mengen von 0,5 bis 3,0 Gew.-% oder Spuren von oligomeren Verbindungen, enthalten.

Nach der Abtrennung zumindest eines Teils der Base kann die Zusammensetzung weiteren Zwischenbehandlungen unterworfen werden.

Vorzugsweise weist das cyclische Olefin im Rahmen der vorliegenden Erfindung mindestens zwei C-C-Doppelbindungen auf, also beispielsweise 2, 3, 4 oder 5.

Daher betrifft die vorliegende Erfindung gemäß einer weiteren Ausführungsform auch ein wie zuvor beschriebenes Verfahren zur Herstellung eines cyclischen Ketons mit 7 bis 16 C-Atomen, wobei das cyclische Olefin mindestens zwei C-C-Doppelbindungen aufweist.

Sofern das cyclische Olefin mehr als eine C-C-Doppelbindung aufweist, wird bei der Oxidation gemäß Stufe (a) des erfindungsgemäßen Verfahrens vorzugsweise nur eine der C-C-Doppelbindungen oxidiert.

Dabei wird erfindungsgemäß die Oxidation gemäß Stufe (a) so durchgeführt, dass bei der Reaktion möglichst wenig, insbesondere möglichst keine Rückvermischung auftritt.

Sofern gemäß Stufe (a) nur eine der C-C-Doppelbindungen des in Zusammensetzung (I) enthaltenen cyclischen Olefins oxidiert wird, ist es im Rahmen der vorliegenden Erfindung möglich, die mindestens eine verbleibende C-C-Doppelbindung des in der Zusammensetzung (A) oder Zusammensetzung (B) enthaltenen cyclischen Ketons nach Stufe (b) in einem weiteren Reaktionsschritt umzusetzten. Vorzugsweise wird die mindestens eine verbleibende C-C-Doppelbindung im Rahmen der vorliegenden Erfindung hydriert.

Erfindungsgemäß kann die in Stufe (b) erhaltene Zusammensetzung (B) gemäß Stufe (c) hydriert werden.

Bei der Hydrierung gemäß Stufe (c) wird das in Zusammensetzung (B) enthaltene cyclische Keton hydriert. Dabei wird eine Zusammensetzung (C) enthaltend ein cyclisches Keton erhalten, wobei dieses cyclische Keton vorzugsweise keine C-C-Doppelbindung mehr aufweist.

Daher betrifft die vorliegende Erfindung gemäß einer weiteren Ausführungsform auch ein wie zuvor beschriebenes Verfahren zur Herstellung eines cyclischen Ketons mit 7 bis 16 C-Atomen, wobei das Verfahren zusätzlich die Stufe (c) aufweist:
(c) Hydrieren der Zusammensetzung (B) in Gegenwart mindestens eines Katalysators unter Erhalt einer Zusammensetzung (C).

Dabei enthält die Zusammensetzung (C) das cyclische Keton üblicherweise in einer Menge von mehr als 80 Gew.%, vorzugsweise 85 bis 99,9 Gew.%, insbesondere 88 bis 99,9 Gew.%, besonders bevorzugt 90 bis 99,6 Gew.%, weiter bevorzugt 92 bis 99,0 Gew.-%. Die Zusammensetzung (C) enthält neben dem cyclischen Keton üblicherweise weitere Verbindungen, insbesondere organische Verbindungen, vorzugsweise solche mit sauerstoffhaltigen Gruppen, beispielsweise Alkohole, Aldehyde oder Epoxide. Dabei können die organischen Verbindungen insbesondere dieselbe Anzahl an C-Atomen aufweisen wie das in der Zusammensetzung (C) enthaltene cyclische Keton.

Die Nebenkomponenten sind in der Zusammensetzung (C) insbesondere zu weniger als 20 Gew.% enthalten, insbesondere weniger als 15 Gew.%, besonders bevorzugt weniger als 12 Gew.%. Beispielsweise sind die Nebenkomponenten in einer Menge von 0,001 bis 10 Gew.-%, insbesondere von 0,1 bis 9 Gew.% enthalten, bevorzugt von 0,5 bis 5 Gew.%, insbesondere bevorzugt von 1 bis 4 Gew.%.

Gemäß der vorliegenden Erfindung kann die Zusammensetzung (B) direkt in die Stufe (c) eingesetzt werden. Es ist jedoch im Rahmen der vorliegenden Erfindung ebenfalls möglich, dass die Zusammensetzung (B) zunächst behandelt wird und dann in Stufe (c) eingesetzt wird.

Für die Hydrierung gemäß Stufe (c) können sämtliche geeigneten Katalysatoren eingesetzt werden. Insbesondere sind mindestens ein homogener oder mindestens ein heterogener oder sowohl mindestens ein homogener und mindestens ein heterogener Katalysator einsetzbar.

Bevorzugt enthalten die einsetzbaren Katalysatoren mindestens ein Metall aus der 7., der 8., der 9., der 10. oder der 11. Nebengruppe des Periodensystems der Elemente. Weiter bevorzugt enthalten die erfindungsgemäß einsetzbaren Katalysatoren mindestens ein Element, ausgewählt aus der Gruppe bestehend aus Re, Fe, Ru, Co, Rh, Ir, Ni, Pd, Pt, Cu und Au. Insbesondere bevorzugt enthalten die erfindungsgemäß einsetzbaren Katalysatoren mindestens ein Element, ausgewählt aus der Gruppe bestehend aus Fe, Ni, Pd, Pt und Cu. Besonders bevorzugt enthalten die erfindungsgemäß einsetzbaren Katalysatoren Pd, Pt, Ru oder Ni.

Geeignet sind beispielsweise homogene Katalysatoren enthaltend mindestens ein Element der 8., 9. oder 10. Nebengruppe. Weiter bevorzugt sind homogene Katalysatoren, die Ru, Rh, Ir und/oder Ni enthalten. Beispielsweise sind hierbei etwa RhCl(TTP)₃ oder R_{U4}H₄(CO)₁₂ zu nennen. Besonders bevorzugt sind solche homogenen Katalysatoren, die Ru enthalten. Beispielsweise werden homogene Katalysatoren eingesetzt, wie sie in der US 5,180,870, US 5,321,176, US 5,177,278, US 3,804,914, US 5,210,349 US 5,128,296, US B 316,917 und in D.R. Fahey in J. Org. Chem. 38 (1973) S. 80-87 beschrieben sind, deren diesbezügliche Offenbarung in den Kontext der vorliegenden Anmeldung vollumfänglich einbezogen wird. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)₂RuH₂, (TPP)₂(CO)₂RuCH oder (TPP)₃(CO)RuCl₂.

Geeignet ist insbesondere mindestens ein heterogener Katalysator, wobei mindestens eines der obenstehend genannten Metalle als Metall als solches, als Raney-Katalysator und/oder aufgebracht auf einen üblichen Träger eingesetzt werden kann. Bevorzugte Trägermaterialien sind etwa Aktivkohlen oder Oxide wie beispielsweise Aluminiumoxide, Siliciumoxide, Titanoxide oder Zirkonoxide. Ebenso sind unter anderem als Trägermaterialien Bentonite zu nennen. Werden zwei oder mehr Metalle eingesetzt, so können diese separat oder als Legierung vorliegen. Hierbei ist es möglich, mindestens ein Metall als solches und mindestens ein anderes Metall als Raney-Katalysator oder mindestens ein Metall als solches und mindestens ein anderes Metall, aufgebracht auf mindestens einen Träger, oder mindestens ein Metall als Raney-Katalysator und mindestens ein anderes Metall, aufgebracht auf mindestens einen Träger, oder mindestens ein Metall als solches und mindestens ein anderes Metall als Raney-Katalysator und mindestens ein anderes Metall, aufgebracht auf mindestens einen Träger, einzusetzen.

Die eingesetzten Katalysatoren können beispielsweise auch so genannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, beispielsweise schwerlöslichen Hydroxiden, Oxidhydraten, basischen Salzen oder Carbonaten ausfällt, die erhaltenen Niederschläge anschließend trocknet und diese dann durch Calcinierung bei im allgemeinen 300 bis 700°C, insbesondere 400 bis 600°C in die entsprechenden Oxide, Mischoxide und/oder gemischtvalentigen Oxide umwandelt, die durch eine Behandlung mit Wasserstoff oder mit Wasserstoff enthaltenden Gasen im Bereich von im Allgemeinen 50 bis 700°C, insbesondere 100 bis 400°C zu den betreffenden Metallen und/oder oxidischen Verbindungen niederer Oxidationsstufe reduziert und in die eigentliche katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft gleichzeitig mit dem Trägermaterial aus den betreffenden Salzlösungen gefällt werden.

Bevorzugt werden Hydrierkatalysatoren eingesetzt, welche die die Hydrierung katalysierenden Metalle oder Metallverbindungen auf einem Trägermaterial abgeschieden enthalten.

Außer den oben genannten Fällungskatalysatoren, die außer den katalytisch aktiven Komponenten noch zusätzlich ein Trägermaterial enthalten, eignen sich für das erfindungsgemäße Verfahren im Allgemeinen solche Trägermaterialien, bei denen die katalytisch-hydrierend wirkende Komponente beispielsweise durch Imprägnierung auf ein Trägermaterial aufgebracht worden ist.

Die Art des Aufbringens des katalytisch aktiven Metalls auf den Träger ist in der Regel nicht kritisch und kann auf verschiedenerlei Art und Weise bewerkstelligt werden. Die katalytisch aktiven Metalle können auf diesen Trägermaterialien beispielsweise durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, beispielsweise mit Nitraten oder thermisch leicht zersetzbaren Komplexverbindungen, beispielsweise Carbonyl- oder Hydrido-Komplexen der katalytisch aktiven Metalle, zu imprägnieren und den so getränkten Träger zur thermischen Zersetzung der adsorbierten Metallverbindungen auf Temperaturen im Bereich von 300 bis 600°C zu erhitzen. Diese thermische Zersetzung wird vorzugsweise unter einer Schutzgasatmosphäre vorgenommen. Geeignete Schutzgase sind beispielsweise Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Im Allgemeinen führen höhere Gehalte an katalytisch aktiven Metallen dieser Trägerkatalysatoren zu höheren Raum-Zeit-Umsätzen als niedrigere Gehalte. Im Allgemeinen werden Trägerkatalysatoren verwendet, deren Gehalt an katalytisch aktiven Metallen im Bereich von 0,1 bis 90 Gew.-%, vorzugsweise im Bereich von 0,5 bis 40 Gew.-% bezogen auf das Gesamtgewicht des Katalysators, liegt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, ist es auch denkbar, dass diese Angaben auch unter- oder überschritten werden können, ohne dass sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-OS 25 19 817, EP 1 477 219 A1 oder EP 0 285 420 A1 auf den Träger aufgebracht werden. In den Katalysatoren gemäß den vorgenannten Schriften liegen die katalytisch aktiven Metalle als Legierungen vor, die durch thermische Behandlung und/oder Reduktion der z.B. durch Tränkung des Trägermaterials mit einem Salz oder Komplex der zuvor genannten Metalle, erzeugt werden.

Sowohl die Aktivierung der Fällungskatalysatoren als auch der Trägerkatalysatoren kann auch in situ zu Beginn der Reaktion durch den anwesenden Wasserstoff erfolgen. Bevorzugt werden diese Katalysatoren vor ihrer Verwendung separat aktiviert.

Als Trägermaterialien können im Allgemeinen die Oxide des Aluminiums und Titans, Zirkoniumdioxid, Siliciumdioxid, Tonerden wie beispielsweise Montmorillonite, Silikate wie beispielsweise Magnesium- oder Aluminiumsilikate, Zeolithe wie beispielsweise der Strukturtypen ZSM-5 oder ZSM-10, oder Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren verwendbare Katalysatoren dienen.

Ganz besonders bevorzugte Katalysatoren sind erfindungsgemäß solche, die Ni, Pt und/oder Pd enthalten und auf einem Träger aufgebracht sind. Ganz bevorzugte Träger sind oder enthalten Aktivkohle, Aluminiumoxid, Titandioxid und/oder Siliziumdioxid.

Der mindestens eine heterogene Katalysator kann beispielsweise als Suspensionskatalysator und/oder als Festbettkatalysator eingesetzt werden.

Wird beispielsweise im Rahmen des erfindungsgemäßen Verfahrens die Hydrierung gemäß Stufe (c) mit mindestens einem Suspensionskatalysator durchgeführt, so wird bevorzugt in mindestens einem Rührreaktor oder in mindestens einer Blasensäule oder in mindestens einer gepackten Blasensäule oder in einer Kombination aus zwei oder mehr gleichen oder unterschiedlichen Reaktoren hydriert.

Der Begriff "unterschiedliche Reaktoren" bezeichnet vorliegend sowohl unterschiedliche Reaktortypen als auch Reaktoren der gleichen Art, die sich beispielsweise durch ihre Geometrie wie beispielsweise ihr Volumen und/oder ihren Querschnitt und/oder durch die Hydrierbedingungen in den Reaktoren unterscheiden.

Wird beispielsweise im Rahmen des erfindungsgemäßen Verfahrens die Hydrierung gemäß Stufe (c) mit mindestens einem fest angeordneten Katalysator durchgeführt, so wird bevorzugt mindestens ein Rohrreaktor wie beispielsweise mindestens ein Schachtreaktor und/oder mindestens ein Rohrbündelreaktor verwendet, wobei ein einzelner Reaktor in Sumpf- oder Rieselfahrweise betrieben werden kann. Bei Verwendung von zwei oder mehr Reaktoren kann mindestens einer in Sumpffahrweise und mindestens einer in Rieselfahrweise betrieben werden.

Wird als Katalysator bei der Hydrierung beispielsweise ein heterogener Katalysator als Suspensionskatalysator eingesetzt, so wird dieser im Rahmen der vorliegenden Erfindung bevorzugt durch mindestens einen Filtrationsschritt abgetrennt. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurück zu gewinnen.

Wird als Katalysator bei der Hydrierung gemäß Stufe (c) beispielsweise ein homogener Katalysator eingesetzt, so wird dieser im Rahmen der vorliegenden Erfindung bevorzugt durch mindestens einen Destillationsschritt abgetrennt. Im Rahmen dieser Destillation können eine oder zwei oder mehr Destillationskolonnen verwendet werden. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurück zu gewinnen.

Vor dem Einsatz in einem beliebigen Verfahren wie beispielsweise vor der Rückführung in das erfindungsgemäße Verfahren kann sowohl der mindestens eine homogene als auch der mindestens eine heterogene Katalysator, sollte dies erforderlich sein, durch mindestens ein geeignetes Verfahren regeneriert werden.

Die Wärme kann bei dem erfindungsgemäß verwendeten Reaktor intern beispielsweise über Kühlschlangen und/oder extern beispielsweise über mindestens einen Wärmetauscher abgeführt werden. Wird beispielsweise bevorzugt mindestens ein Rohrreaktor zur Hydrierung eingesetzt, so wird bevorzugt die Reaktion über äußeren Umlauf gefahren, in dem die Wärmeabfuhr integriert ist.

Wird gemäß einer bevorzugten Ausführungsform des erfindungsgemäßen Verfahrens die Hydrierung kontinuierlich durchgeführt, werden weiter bevorzugt mindestens zwei Reaktoren, weiter bevorzugt mindestens zwei Rohrreaktoren, weiter bevorzugt mindestens zwei seriell gekoppelte Rohrreaktoren und insbesondere bevorzugt genau zwei seriell gekoppelte Rohrreaktoren eingesetzt. Die Hydrierbedingungen in den eingesetzten Reaktoren können jeweils gleich oder unterschiedlich sein und liegen jeweils in den oben beschriebenen Bereichen.

Wird die Hydrierung gemäß Stufe (c) an mindestens einem suspendierten Katalysator durchgeführt, liegt die Verweilzeit im Allgemeinen im Bereich von 0,05 bis 50 h, beispielsweise im Bereich von 0,5 bis 50 h, bevorzugt im Bereich von 1 bis 30 h und besonders bevorzugt im Bereich von 1,5 bis 25 h, ganz besonders bevorzugt im Bereich von 1,5 bis 10 h. Dabei ist es unerheblich, ob erfindungsgemäß ein Hauptreaktor und ein Nachreaktor oder zusätzlich weitere Reaktoren eingesetzt werden. Für sämtliche dieser Ausführungsformen liegt die Gesamtverweilzeit in den oben angegebenen Bereichen.

Wird im Rahmen des erfindungsgemäßen Verfahrens die Hydrierung in kontinuierlicher Fahrweise an mindestens einem fest angeordneten Katalysator durchgeführt, so liegt die Katalysatorbelastung (kg Feed/Liter Katalysator x h) im Allgemeinen im Bereich von 0,03 bis 20, bevorzugt im Bereich von 0,05 bis 5 und besonders bevorzugt im Bereich von 0,1 bis 2. Dabei ist es unerheblich, ob erfindungsgemäß ein Hauptreaktor und ein Nachreaktor oder zusätzlich weitere Reaktoren eingesetzt werden. Für sämtliche dieser Ausführungsformen liegt die Gesamtbelastung in den oben angegebenen Bereichen.

Allgemein liegt die Hydriertemperatur im Hauptreaktor im Bereich von 0 bis 350°C, bevorzugt im Bereich von 20 bis 300°C, weiter bevorzugt im Bereich von 50 bis 250°C und insbesondere bevorzugt im Bereich von 80 bis 220°C.

Der Wasserstoffdruck liegt bei der erfindungsgemäßen Hydrierung im Hauptreaktor im Allgemeinen im Bereich von 1 bis 325 bar, bevorzugt im Bereich von 5 bis 300 bar, weiter bevorzugt im Bereich von 10 bis 250 bar und insbesondere bevorzugt im Bereich von 15 bis 150 bar.

Im Rahmen der erfindungsgemäßen Hydrierung gemäß Stufe (c) kann mindestens ein geeignetes Lösungsmittel oder Verdünnungsmittel eingesetzt werden. Als solche sind grundsätzlich sämtliche Lösungs- und Verdünnungsmittel zu nennen, die unter den Hydrierbedingungen nicht hydriert oder anderweitig umgesetzt werden, z.B. Alkohole, Ether, Kohlenwasserstoffe, Wasser, Aromaten oder Ketone, insbesondere Toluol oder Cyclododecan.

Gemäß einer bevorzugten Ausführungsform des erfindungsgemäßen Verfahrens wird die Hydrierung gemäß Stufe (c) ohne Zusatz eines Lösungs- oder Verdünnungsmittels durchgeführt.

Im Rahmen der vorliegenden Erfindung ist es möglich, dass zwischen den Stufen (a) und (b) oder zwischen den Stufen (b) und (c) oder zwischen den Stufen (a) und (b) und (b) und (c) mindestens eine Zwischenbehandlung erfolgt. Dabei ist es beispielsweise möglich, dass im Rahmen der Zwischenbehandlung nicht umgesetztes Edukt oder Nebenprodukte ganz oder teilweise entfernt werden. Sofern im Anschluß an die Stufe (a) eine Zwischenbehandlung der Zusammensetzung (A) erfolgt, wird erfindungsgemäß eine Zusammensetzung (A') erhalten, die dann in die Stufe (b) eingesetzt wird. Ebenso ist es erfindungsgemäß möglich, dass im Anschluß an die Stufe (b) eine Zwischenbehandlung der Zusammensetzung (B) erfolgt, wobei dann erfindungsgemäß eine Zusammensetzung (B') erhalten, die dann in die Stufe (c) eingesetzt wird.

Daher betrifft die vorliegende Erfindung gemäß einer weiteren Ausführungsform auch ein wie zuvor beschriebenes Verfahren zur Herstellung eines cyclischen Ketons mit 7 bis 16 C-Atomen, wobei nach Stufe (a) und vor Stufe (b) nicht umgesetztes Edukt oder Nebenprodukte aus der Zusammensetzung (A) unter Erhalt einer Zusammensetzung (A') entfernt werden.

Ebenso ist es im Rahmen der Erfindung möglich, dass eine Zwischenbehandlung mehrere verschiedene Schritte umfasst, die gleich oder verschieden sein können.

Als weitere mögliche Zwischenbehandlungen sind etwa zu nennen:
- Erwärmung des Reaktionsgutes;
- Änderung des Druckes, unter dem sich das Reaktionsgut befindet. Bevorzugt ist in diesem Zusammenhang beispielsweise die Erhöhung des Druckes über beispielsweise mindestens eine Pumpe und/oder mindestens einen Kompressor;
- Zudosierung mindestens eines Edukts. Insbesondere kann Lösungsmittel zudosiert werden.
- Abtrennung von gebildetem Produkt durch mindestens eine geeignete Maßnahme wie beispielsweise bevorzugt durch mindestens einen destillativen Schritt.
- Abtrennung von nicht umgesetztem Edukt durch mindestens eine geeignete Maßnahme wie beispielsweise bevorzugt durch mindestens einen destillativen Schritt

Im Rahmen der vorliegenden Erfindung kann das erfindungsgemäße Verfahren nach der Stufe (c) weitere Schritte umfassen, beispielsweise Reinigungsschritte oder Schritte zur Abtrennung unerwünschter Nebenprodukte.

Dabei kann nach der Stufe (c) beispielsweise thermisches Behandeln der Zusammensetzung (C) mit mindestens einer Säure oder mindestens einem Katalysator, der mindestens ein Übergangsmetall enthält, erfolgen oder weiteres Aufreinigen durch ein Verfahren ausgewählt aus der Gruppe bestehend aus Destillation, Extraktion und Kristallisation.

Erfindungsgemäß wird jedoch nach der Hydrierung gemäß Stufe (c) keine Behandlung der erhaltenen Zusammensetzung (C) durchgeführt, die die Schritte
(i) thermisches Behandeln der Zusammensetzung (C) mit mindestens einer Säure oder mindestens einem Katalysator, der mindestens ein Übergangsmetall enthält, und
(ii) weiteres Aufreinigen durch ein Verfahren ausgewählt aus der Gruppe bestehend aus Destillation, Extraktion und Kristallisation
umfasst.

Gemäß einer bevorzugten Ausführungsform der vorliegenden Erfindung erfolgt nach der Stufe (c) eine destillative Aufreinigung der Zusammensetzung (C).

Daher betrifft die vorliegende Erfindung gemäß einer weiteren Ausführungsform auch ein wie zuvor beschriebenes Verfahren zur Herstellung eines cyclischen Ketons mit 7 bis 16 C-Atomen, wobei das Verfahren nach der Stufe (c) noch eine destillative Aufreinigung umfasst.

Im Rahmen einer bevorzugten Ausführungsform der vorliegenden Erfindung wird als in Zusammensetzung (I) enthaltenes cyclisches Olefin Cyclododecatrien eingesetzt, bevorzugt 1,5,9-Cyclododecatrien.

Daher betrifft die vorliegende Erfindung gemäß einer weiteren Ausführungsform auch ein wie zuvor beschriebenes Verfahren zur Herstellung eines cyclischen Ketons mit 7 bis 16 C-Atomen, wobei das cyclische Olefin Cyclododecatrien ist.

Sofern Cyclododecatrien eingesetzt wird, ist es im Rahmen der vorliegenden Erfindung bevorzugt, dass das erfindunsgemäße Verfahren auch die Stufe (c) umfasst.

Vorzugsweise wird Cyclododecatrien durch Trimerisierung von Butadien erhalten.

Daher betrifft die vorliegende Erfindung gemäß einer weiteren Ausführungsform auch ein wie zuvor beschriebenes Verfahren zur Herstellung eines cyclischen Ketons mit 7 bis 16 C-Atomen, wobei das cyclische Olefin Cyclododecatrien ist, das mittels Trimerisierung aus Butadien hergestellt wurde.

1,5,9-Cyclododecatrien kann beispielsweise durch Trimerisierung von reinem 1,3-Butadien hergestellt werden, wie dies beispielsweise in T. Schiffer, G. Oenbrink, "Cyclododecatriene, Cyclooctadiene, and 4-Vinylcyclohexene", Ullmann's Encyclopedia of Industrial Chemistry, 6th Edition (2000), Electronic Release, Wiley VCH beschrieben ist. Im Rahmen dieses Verfahrens entstehen beispielsweise bei der Trimerisierung in Anwesenheit von Ziegler-Katalysatoren cis,trans,trans-1,5,9-Cyclododecatrien, cis,cis,trans-1,5,9-Cyclododecatrien und all-trans-1,5,9-Cyclododecatrien, wie dies beispielsweise in H. Weber et al. "Zur Bildungsweise von cis,trans,trans-Cyclododecatrien-(1.5.9) mittels titanhaltiger Katalysatoren" in: Liebigs Ann. Chem. 681 (1965) S.10-20 beschrieben ist. Cyclododecatrien kann durch Trimerisierung von 1,3-Butadien unter Verwendung eines Titan-Katalysators hergestellt werden.

Während grundsätzlich sämtliche geeigneten Titan-Katalysatoren zur Trimerisierung eingesetzt werden können, ist der im Artikel von Weber et al. beschriebene Titantetrachlorid/Ethylaluminiumsesquichlorid-Katalysator besonders geeignet.

Das für die Trimerisierung eingesetzte Butadien weist insbesondere bevorzugt einen über Gaschromatographie bestimmten Reinheitsgrad von mindestens 99,6 % und weiter bevorzugt von mindestens 99,65 % auf. Insbesondere bevorzugt enthält das eingesetzte 1,3-Butadien im Rahmen der Nachweisgenauigkeit kein 1,2-Butadien und kein 2-Butin.

Aus dieser Trimerisierung werden im Allgemeinen Gemische erhalten, die mindestens 95 Gew.-%, bevorzugt mindestens 96 Gew.-% und weiter bevorzugt mindestens 97 Gew.-% cis,trans,trans-1,5,9-Cyclododecatrien enthalten. Beispielsweise insbesondere bevorzugt enthalten die Gemische in etwa 98 Gew.-% cis,trans,trans-1,5,9-Cyclododecatrien.

Dieses cis,trans,trans-1,5,9-Cyclododecatrien enthaltende Gemisch kann als solches für die Umsetzung gemäß Stufe (a) verwendet werden. Ebenso ist es möglich, über mindestens eine geeignete Methode, beispielsweise bevorzugt über mindestens eine Destillation, das cis,trans,trans-1,5,9-Cyclododecatrien aus dem Gemisch abzutrennen und in der Umsetzung gemäß Stufe (a) einzusetzen.

Im Rahmen der vorliegenden Erfindung kann gemäß Stufe (a) grundsätzlich jedes Cyclododecatrien oder jedes Gemisch aus zwei und mehr unterschiedlichen Cyclododecatrienen mit Distickstoffmonoxid umgesetzt werden. Unter anderem sind hierbei beispielsweise 1,5,9-Cyclododecatriene, beispielsweise cis,trans,trans-1,5,9-Cyclododecatrien oder cis,cis,trans-1,5,9-Cyclododecatrien oder all-trans-1,5,9-Cyclododecatrien, zu nennen.

Gemäß einer ganz besonders bevorzugten Ausführungsform des erfindungsgemäßen Verfahrens wird als Cyclododecatrien cis,trans,trans-1,5,9-Cyclododecatrien eingesetzt.

Gemäß einer ganz besonders bevorzugten Ausführungsform des erfindungsgemäßen Verfahrens wird als Cyclododecatrien ein Isomerengemisch eingesetzt, das überwiegend cis,trans,trans-1,5,9-Cyclododecatrien, trans,trans,trans-1,5,9-Cyclododecatrien oder cis,cis,trans-1,5,9-Cyclododecatrien enthält. Vorzugsweise wird ein Isomerengemisch eingesetzt, das mehr als 60 Gew.-%, bezogen auf das Isomerengemisch, cis,trans,trans-1,5,9-Cyclododecatrien enthält, weiter bevorzugt mehr als 70 Gew.-%, insbesondere mehr als 80 Gew.-%, besonders bevorzugt mehr als 90 Gew.-%, beispielsweise mehr als 91 Gew.-%, mehr als 92 Gew.-%, mehr als 93 Gew.-%, mehr als 94 Gew.-%, mehr als 95 Gew.-%, mehr als 96 Gew.-%, mehr als 97 Gew.-% oder mehr als 98 Gew.-%.

Im allgemeinen resultiert aus der erfindungsgemäßen Umsetzung von cis,trans,trans-1,5,9-Cyclododecatrien mit Distickstoffmonoxid gemäß Stufe (a) ein Cyclododeca-4,8-dienon-Isomerengemisch, das mindestens zwei der Isomere cis,trans-Cyclododeca-4,8-dienon, trans,cis-Cyclododeca-4,8-dienon und trans,trans-Cyclododeca-4,8-dienon enthält. Bevorzugt wird erfindungsgemäß ein Isomerengemisch erhalten, bei dem trans,cis-Isomer und cis,trans-Isomer in etwa in gleichen Mengen gebildet werden und das trans,trans-Isomer im Vergleich zu den beiden anderen Isomeren in nur geringen Mengen gebildet wird. Ein beispielsweise typisches Isomerengemisch weist demgemäß die Isomeren in molaren Verhältnissen von in etwa 1 : 1 : 0,08 auf.

Aus der erfindungsgemäßen Umsetzung von Zusammensetzung (I) enthaltend Cyclododecatrien mit Distickstoffmonoxid gemäß Stufe (a) wird im Allgemeinen als Zusammensetzung (A) ein Gemisch erhalten, das Cyclododecadienon, bevorzugt Cyclododeca-4,8-dienon, und gegebenenfalls nicht umgesetztes Edukt und/oder gegebenenfalls mindestens ein Nebenprodukt enthält. Je nach weiterer Verwertung und/oder Aufarbeitung kann aus diesem Gemisch das Cyclododecadienon, bevorzugt das Cyclododeca-4,8-dienon abgetrennt werden.

Aus diesem Gemisch kann das Cyclododeca-4,8-dienon durch mindestens eine geeignete Methode abgetrennt werden. Bevorzugt ist hierbei die destillative Abtrennung. Die Destillation erfolgt hierbei bei einem Druck im Bereich von im Allgemeinen 0,001 bis 2 bar, bevorzugt im Bereich von 0,01 bis 1 bar und insbesondere bevorzugt im Bereich von 0,02 bis 0,5 bar.

Gemäß einer besonders bevorzugten Ausführungsform des erfindungsgemäßen Verfahrens wird gemäß Stufe (c) eine Zusammensetzung (B) enthaltend Cyclododeca-4,8-dienon zu einer Zusammensetzung (C) enthaltend Cyclododecanon hydriert.

Gemäß einer weiteren bevorzugten Ausführungsform der Erfindung wird gemäß Stufe (a) eine Zusammensetzung (I) enthaltend Cyclododecen eingesetzt. In diesem Fall umfasst das erfindungsgemäße Verfahren vorzugsweise keine Stufe (c).

Aus der erfindungsgemäßen Oxidation des Cyclododecens gemäß Stufe (a) resultiert im Allgemeinen ein Produktgemisch. Bevorzugt enthält dieses Produktgemisch im Bereich von 5 bis 95 Gew.-%, besonders bevorzugt von 7 bis 80 Gew.-% und insbesondere bevorzugt von 10 bis 75 Gew.-% Cyclododecanon, jeweils bezogen auf das Gesamtgewicht des Produktgemisches nach Abkühlung auf 20 °C und Entspannung auf Normaldruck..

Das als Edukt eingesetzte Cyclododecen, das entweder als cis-Isomer oder als trans-Isomer oder als Gemisch aus cis- und trans-Isomer eingesetzt werden kann, kann grundsätzlich aus jeder beliebigen Quelle stammen.

Ganz besonders bevorzugt wird im Rahmen der vorliegenden Erfindung Cyclododecen durch Partialhydrierung mindestens eines Cyclododecatriens, bevorzugt durch Partialhydrierung mindestens eines 1,5,9-Cyclododecatriens wie beispielsweise cis,trans,trans-1,5,9-Cyclododecatrien oder cis,cis,trans-1,5,9-Cyclododecatrien oder all-trans-1,5,9-Cyclododecatrien und insbesondere aus cis,trans,trans-1,5,9-Cyclododecatrien hergestellt.

Gemäß einer besonders bevorzugten Ausführungsform der vorliegenden Erfindung werden die Stufen (a) und (b) und gegebenenfalls auch (c) des erfindungsgemäßen Verfahrens mit mehreren Zwischenbehandlungsstufen kombiniert.

Bevorzugte Ausführungsformen der vorliegenden Erfindung sind im folgenden beispielhaft wiedergegeben, wobei das erfindungsgemäße Verfahren nicht auf die angegebenen Ausführungsformen beschränkt ist.

Gemäß einer bevorzugten Ausführungsform des erfindungsgemäßen Verfahrens wird demgemäß gemäß Stufe (a) eine Zusammensetzung (I) enthaltend 1,5,9-Cyclododecatrien mit Distickstoffmonoxid oxidiert wobei als Zusammensetzung (A) ein Gemisch erhalten wird, das neben unumgesetztem Cyclododecatrien noch Cyclododeca-4,8-dienon als Hauptprodukt und Dodeca-4,8,11-trienal, Cycloundeca-3,7-diencarbaldehyd und Cyclododecendione als Nebenprodukte enthält.

Aus diesem Gemisch wird das Cyclododecatrien abgetrennt, vorzugsweise durch Destillation, wobei man ein Gemisch enthält das im wesentlichen aus Cyclododeca-4,8-dienon besteht mit Dodeca-4,8,11-trienal, Cycloundeca-3,7-diencarbaldehyd und Cyclododecendionen als Nebenkomponenten. Vorzugsweise wird Dodeca-4,8,11-trienal durch Destillation abgereichert. Anschließend werden vorzugsweise die Cyclododecendione abgetrennt, vorzugsweise durch Destillation, um als Zusammensetzung (A') ein Gemisch zu erhalten, das im wesentlichen Cyclododeca-4,8-dienon und Cycloundeca-3,7-diencarbaldehyd mit kleinen Beimengungen an Dodeca-4,8,11-trienal enthält.

Gemäß Stufe (b) des erfindungsgemäßen Verfahrens wird dann vorzugsweise die so erhaltene Zusammensetzung (A') mit Base versetzt und behandelt, bis der gewünschte Abreicherungsgrad an Aldehyden erreicht ist. Nach dem Behandeln wird die Base abgetrennt, vorzugsweise durch eine Destillation. Die Destillation kann beispielsweise über eine Trennwandkolonne erfolgen.

Die dabei erhaltene Zusammensetzung (B) kann dann vorzugsweise gemäß Stufe (c) hydriert werden, um eine Zusammensetzung (C) zu erhalten, die im wesentlichen aus Cyclododecanon besteht. An die Stufe (c) kann eine weitere Feinreinigung anschließen, beispielsweise eine Destillation.

Vorzugsweise wird gemäß der zuvor beschriebenen bevorzugten Ausführungsform die Abtrennung der Nebenprodukte aus Zusammensetzung (A) durch Destillation erreicht. Dabei wird gemäß einer besonders bevorzugten Ausführungsform des Verfahrens in einer ersten Kolonne Cyclododecatrien über Kopf abdestilliert und im Sumpf ein Gemisch enthaltend Cyclododeca-4,8-dienon, Dodeca-4,8,11-trienal, Cycloundeca-3,7-diencarbaldehyd und Cyclododecendione erhalten. In einer zweiten Kolonne wird das Sumpfprodukt vorzugsweise in einer Kopffraktion, die im wesentlichen Dodeca-4,8,11-trienal enthält, und einer Sumpffraktion, die die anderen Komponenten enthält, aufgetrennt. Dieser Sumpfaustrag wird dann bevorzugt in einer dritte Kolonne weiter aufgetrennt, wobei man als Kopfprodukt ein Gemisch aus im wesentlichen Cyclododeca-4,8-dienon und Cycloundeca-3,7-diencarbaldehyd erhält und als Sumpf im wesentlichen Cyclododecendione. Das Kopfprodukt der letzten Kolonne wird dann erfindungsgemäß als Zusammensetzung (A') gemäß Stufe (b) mit einer Base behandelt.

In einer weiteren bevorzugten Ausführungsform des Verfahrens wird die Zwischenbehandlung derart durchgeführt, dass in einer ersten Kolonne Cyclododecatrien über Kopf abdestilliert wird und im Sumpf ein Gemisch enthaltend Cyclododeca-4,8-dienon, Dodeca-4,8,11-trienal, Cycloundeca-3,7-diencarbaldehyd und Cyclododecendione erhalten wird. In einer zweiten Kolonne wird das Sumpfprodukt in eine Kopffraktion, die im wesentlichen Dodeca-4,8,11-trienal enthält, eine Sumpffraktion, die im wesentlichen Cyclododecendione enthält, und in eine Seitenabzugfraktion, die im wesentlichen Cyclododeca-4,8-dienon und Cycloundeca-3,7-diencarbaldehyd enthält, aufgetrennt. Die Seitenabzugsfraktion der zweiten Kolonne wird dann erfindungsgemäß als Zusammensetzung (A') gemäß Stufe (b) mit einer Base behandelt.

Erfindungsgemäß ist es jedoch auch möglich, die einzelnen Schritte der Zwischenbehandlung in unterschiedlicher Reihenfolge durchzuführen bzw. nicht alle oben genannten Trennschritte durchzuführen.

Insbesondere kann das gemäß Stufe (a) erhaltene Produkt direkt gemäß Stufe (b) mit einer Base behandelt werden.

Im Folgenden wird die vorliegende Erfindung anhand von Beispielen naher erläutert.

### Beispiele

### Beispiel 1:

1,5,9-Cyclododecatrien wurde gemäß Beispiel 7 der WO 2005/030690 mit N₂O oxidiert und aufgearbeitet. Es wurde so destilliert, dass das Kopfprodukt der zweiten Destillationskolonne folgende Zusammensetzung hatte: Cyclododeca-4,8-dienon (98 Gew.-%), Dodeca-4,8,11-trienal (0,2 Gew.-%) und Cycloundeca-3,7-diencarbaldehyd (0,7 Gew.-%).

500 g des Kopfprodukts wurden in einen Rührkolben und unter Schutzgasatmosphäre (N₂) auf 160°C aufgeheizt. Anschließend wurden mit einer Spritze 5,0 g einer 25 %-igen wässrigen NaOH-Lösung zugegeben. Die Reaktionsmischung blieb klar und homogen. In regelmäßigen Abständen wurden Proben entnommen und mit GC analysiert. Nach 35 min enthielt die Lösung weniger als 20 Gew.-ppm Dodeca-4,8,11-trienal und nur noch ca. 0,2 Gew.-% Cycloundeca-3,7-diencarbaldehyd. Nach 95 min enthielt die Lösung weniger als 10 Gew.-ppm Dodeca-4,8,11-trienal und 400 Gew.-ppm Cycloundeca-3,7-diencarbaldehyd. Der Gehalt an Cyclododeca-4,8-dienon veränderte sich dagegen kaum (97 Gew.-%).

### Beispiel 2:

1,5,9-Cyclododecatrien wurde gemäß Beispiel 7 der WO 2005/030690 mit N₂O oxidiert und wie beschrieben wurde in einer ersten Kolonne nicht umgesetztes 1,5,9-Cyclododecatrien abgetrennt. Der Sumpfaustrag aus der ersten Kolonne hatte folgende Zusammensetzung: Cyclododeca-4,8-dienon (90 Gew.-%), Dodeca-4,8,11-trienal (2,2 Gew.-%), Cycloundeca-3,7-diencarbaldehyd (0,9 Gew.-%) und Cyclododecendione (2,1 Gew.-%). 550 g dieses Gemisches wurden in einem Rührkolben vorgelegt und unter Schutzgas (N₂) auf 160°C aufgeheizt. Anschließend wurden mit einer Spritze 2,75 g einer 25 %-ige wässrigen NaOH-Lösung zugegeben. Die Reaktionsmischung blieb dabei klar und homogen. In regelmäßigen Abständen wurden Proben entnommen und mit GC analysiert. Nach 35 min enthielt die Lösung nur noch etwa 150 Gew.-ppm Dodeca-4,8,11-trienal und 0,4 Gew.-% Cycloundeca-3,7-diencarbaldehyd. Nach 95 min enthielt die Lösung nur noch weniger als 30 Gew.-ppm Dodeca-4,8,11-trienal und 760 Gew.-ppm Cycloundeca-3,7-diencarbaldehyd. Der Gehalt an Cyclododeca-4,8-dienon veränderte sich kaum (88 Gew.-% nach 95 min).

Im Gaschromatogramm waren die den Cyclododecendionen zugeordneten Signale fast vollständig verschwunden. Bei einer etwas geringeren Retentionszeit erschienen im Chromatogramm neue Signale, die einer Molmasse vom 176 g/mol zugeordnet werden.

### Beispiel 3:

Das gemäß Beispiel 2 erhaltene Gemisch wurde in einem Kurzwegverdampfer von hochsiedenen Komponenten befreit. Es wurden etwa 500 g Destillat erhalten. Die 500 g des Destillats wurden in einem 1 Liter Autoklav an 50 ml eines 5 % Pd/Aktivkohlekatalysators bei ca. 120 °C und 30 bar Wasserstoffdruck innerhalb von 5 Stunden hydriert. Der Katalysator wurde anschließend durch Filtration bei ca. 65 °C vom Hydrierprodukt abgetrennt, welches anschließend über eine 1 m Füllkörperkolonne mit 5 mm Metallgewebsringen als Füllkörper fraktioniert bei 10 mbar (absolut) destilliert wurde. Es wurden 85 % des eingesetzten Destillationsedukts als Cyclododecanon mit einer Reinheit von 99,8 % erhalten. Als Nebenkomponenten waren 0,03 % Cycloundecancarbaldehyd und 0,01 % Dodecanol enthalten.

## Patentansprüche

1. Verfahren zur Herstellung eines cyclischen Ketons mit 7 bis 16 C-Atomen, mindestens umfassend die Stufen
(a) Oxidation einer Zusammensetzung (I), mindestens enthaltend ein cyclisches Olefin mit 7 bis 16 C-Atomen mit mindestens einer C-C-Doppelbindung, mittels Distickstoffmonoxid unter Erhalt einer Zusammensetzung (A),
(b) Behandeln der Zusammensetzung (A) mit mindestens einer Base unter Erhalt einer Zusammensetzung (B).

2. Verfahren nach Anspruch 1, wobei die Stufe (b) die Teilschritte (b1) und (b2) umfasst:
(b1) Behandeln der Zusammensetzung (A) mit mindestens einer Base
(b2) Abtrennung der Base.

3. Verfahren nach einem der Ansprüche 1 oder 2, wobei die Base ausgewählt ist aus Natriumhydroxid und Kaliumhydroxid.

4. Verfahren nach einem der Ansprüche 1 bis 3, wobei die Behandlung gemäß Stufe (b) bei einer Temperatur von 100 bis 250°C und für einen Zeitraum von 1 Minute bis 10 Stunden erfolgt.

5. Verfahren nach einem der Ansprüche 1 bis 4, wobei das cyclische Olefin mindestens zwei C-C-Doppelbindungen aufweist.

6. Verfahren nach einem der Ansprüche 1 bis 5, wobei das Verfahren zusätzlich die Stufe (c) aufweist:
(c) Hydrieren der Zusammensetzung (B) in Gegenwart mindestens eines Katalysators unter Erhalt einer Zusammensetzung (C).

7. Verfahren nach einem der Ansprüche 1 bis 6, wobei nach Stufe (a) und vor Stufe (b) nicht umgesetztes Edukt oder Nebenprodukte aus der Zusammensetzung (A) unter Erhalt einer Zusammensetzung (A') entfernt werden.

8. Verfahren nach einem der Ansprüche 6 oder 7, wobei das Verfahren nach der Stufe (c) noch eine destillative Aufreinigung umfasst.

9. Verfahren nach einem der Ansprüche 1 bis 8, wobei das cyclische Olefin Cyclododecatrien ist.

10. Verfahren nach einem der Ansprüche 1 bis 9, wobei das cyclische Olefin Cyclododecatrien ist, das mittels Trimerisierung aus Butadien hergestellt wurde.

11. Verfahren nach einem der Ansprüche 1 bis 8, wobei das cyclische Olefin Cyclododecen ist.

## Claims

1. A process for preparing a cyclic ketone having from 7 to 16 carbon atoms, which comprises at least the steps
(a) oxidation of a composition (I) comprising at least one cyclic olefin which has from 7 to 16 carbon atoms and at least one C-C double bond by means of dinitrogen monoxide to give a composition (A),
(b) treatment of the composition (A) with at least one base to give a composition (B).

2. The process according to claim 1, wherein the step (b) comprises the substeps (b1) and (b2):
(b1) treatment of the composition (A) with at least one base
(b2) removal of the base

3. The process according to claim 1 or 2, wherein the base is selected from among sodium hydroxide and potassium hydroxide.

4. The process according to any of claims 1 to 3, wherein the treatment in step (b) is carried out at a temperature of from 100 to 250°C for a period of from 1 minute to 10 hours.

5. The process according to any of claims 1 to 4, wherein the cyclic olefin has at least two C-C double bonds.

6. The process according to any of claims 1 to 5 which additionally comprises the step (c):
(c) hydrogenation of the composition (B) in the presence of at least one catalyst to give a composition (C).

7. The process according to any of claims 1 to 6, wherein unreacted starting material or by-products are removed from the composition (A) after step (a) and before step (b) to give a composition (A').

8. The process according to claim 6 or 7 which further comprises a purification by distillation after step (c).

9. The process according to any of claims 1 to 8, wherein the cyclic olefin is cyclododecatriene.

10. The process according to any of claims 1 to 9, wherein the cyclic olefin is cyclododecatriene which has been prepared from butadiene by means of trimerization.

11. The process according to any of claims 1 to 8, wherein the cyclic olefin is cyclododecene.

## Revendications

1. Procédé pour la préparation d'une cétone cyclique comprenant 7 à 16 atomes de carbone, comprenant au moins les étapes
(a) oxydation d'une composition (I), contenant au moins une oléfine cyclique comprenant 7 à 16 atomes de carbone avec au moins une double liaison C-C au moyen de protoxyde d'azote avec obtention d'une composition (A),
(b) traitement de la composition (A) au moyen d'une base avec obtention d'une composition (B).

2. Procédé selon la revendication 1, où l'étape (b) comprend les étapes partielles (b1) et (b2) :
(b1) traitement de la composition (A) avec au moins une base
(b2) séparation de la base.

3. Procédé selon l'une quelconque des revendications 1 ou 2, où la base est choisie parmi l'hydroxyde de sodium et l'hydroxyde de potassium.

4. Procédé selon l'une quelconque des revendications 1 à 3, où le traitement selon l'étape (b) est réalisé à une température de 100 à 250°C et pendant un laps de temps de 1 minute à 10 heures.

5. Procédé selon l'une quelconque des revendications 1 à 4, où l'oléfine cyclique présente au moins deux doubles liaisons C-C.

6. Procédé selon l'une quelconque des revendications 1 à 5, où le procédé présente en outre l'étape (c) :
(c) hydrogénation de la composition (B) en présence d'au moins un catalyseur avec obtention d'une composition (C).

7. Procédé selon l'une quelconque des revendications 1 à 6, où, après l'étape (a) et avant l'étape (b) du produit de départ non transformé ou des produits secondaires sont éliminés de la composition (A) avec obtention d'une composition (A').

8. Procédé selon l'une quelconque des revendications 6 ou 7, où le procédé comprend encore une purification par distillation après l'étape (c).

9. Procédé selon l'une quelconque des revendications 1 à 8, où l'oléfine cyclique est le cyclododécatriène.

10. Procédé selon l'une quelconque des revendications 1 à 9, où l'oléfine cyclique est le cyclododécatriène qui a été préparé au moyen d'une trimérisation à partir de butadiène.

11. Procédé selon l'une quelconque des revendications 1 à 8, où l'oléfine cyclique est le cyclododécène.
